# EUROPÄISCHE PATENTSCHRIFT

(11) **EP 3 632 965 B1**
(45) Veröffentlichungstag und Bekanntmachung des Hinweises auf die Patenterteilung: **17.09.2025**
(21) Anmeldenummer: 19210944.5
(22) Anmeldetag: 02.03.2017
(51) Int. Cl.: C08J 5/02, C08J 3/26, B29C 41/14, A61B 42/10

(54) **VERFAHREN ZUM HERSTELLEN EINES PROPHYLAXEARTIKELS**
METHOD FOR PRODUCING A PROPHYLACTIC ITEM
PROCÉDÉ DE FABRICATION D'UN ARTICLE DE TRAITEMENT PROPHYLACTIQUE

(30) Priorität: 04.03.2016 AT 501762016
(43) Veröffentlichungstag der Anmeldung: 08.04.2020
(62) Teilanmeldung aus: 17717060.2
(73) Patentinhaber: Latexx Manufacturing SDN. BHD., 34600 Kamunting (MY)
(72) Erfinder: HOLZNER, Armin, 2630 Ternitz (AT); KERN, Wolfgang, 8055 Seiersberg (AT); MANHART, Jakob Cornelius, 2372 Giesshübl (AT); SAHIN, Melahat, 8700 Leoben (AT); SCHALLER, Raimund, 2620 Neunkirchen (AT); SCHLÖGL, Sandra, 8152 Stallhofen (AT)
(74) Vertreter: Schaad, Balass, Menzl & Partner AG

(56) Entgegenhaltungen:
- EP-A1- 1 209 186
- EP-A1- 2 719 720
- EP-A2- 1 762 586
- WO-A1-00/11980
- WO-A1-2010/105283
- DIETMAR LENKO ET AL: "Dual crosslinking of carboxylated nitrile butadiene rubber latex employing the thiol-ene photoreaction", JOURNAL OF APPLIED POLYMER SCIENCE, vol. 129, no. 5, 5 September 2013 (2013-09-05), US, pages 2735 - 2743, XP055387216, ISSN: 0021-8995, DOI: 10.1002/app.38983
- SANDRA SCHLÖGL ET AL: "Photo-vulcanization using thiol-ene chemistry: Film formation, morphology and network characteristics of UV crosslinked rubber latices", POLYMER., vol. 55, no. 22, 10 June 2014 (2014-06-10), GB, pages 5584 - 5595, XP055387227, ISSN: 0032-3861, DOI: 10.1016/j.polymer.2014.06.007
- BROWN H P ET AL: "CROSSLINKING REACTIONS OF CARBOXYLIC ELASTOMERS", RUBBER CHEMISTRY AND TECHNOLOGY, AMERICAN CHEMICAL SOCIETY, RUBBER DIVISION, US, vol. 36, no. 4, 4 September 1963 (1963-09-04), pages 931 - 962, XP001109664, ISSN: 0035-9475

## Beschreibung

Die Erfindung betrifft ein Verfahren zum Herstellen eines Prophylaxeartikels, insbesondere eines Handschuhs, aus einem (carboxylierten) Dienkautschuk, nach dem auf eine Form zumindest eine Schicht aus einem (carboxylierten) Dienlatex aufgebracht wird, und der (carboxylierte) Dienlatex mit einem Vernetzungsmittel vernetzt wird.

Zudem betrifft die Erfindung die Verwendung eines mehrfach funktionellen Monomers und/oder Polymers.

Prophylaxeartikel, wie insbesondere Operations- und Untersuchungshandschuhe, werden üblicherweise aus einem Elastomerlatex durch eintauchen von handförmigen Tauchformen hergestellt. Auf den Tauchformen bildet sich ein Film, aus dem in weiterer Folge der fertige Einweghandschuh durch Vulkanisation bzw. Vernetzung des Latex entsteht.

Prophylaxeartikel aus Naturlatex weisen ein relativ hohes Allergiepotential auf. Aus diesem Grund werden vermehrt Syntheselatices zur Herstellung der Prophylaxeartikel verwendet. Aber auch diese sind nicht gänzlich hypoallergen, da sie nach wie vor Allergene aus dem Herstellungsprozess aufweisen können, wie beispielsweise Puder zur Verbesserung der Anziehbarkeit, oder Prozesschemikalien, wie beispielsweise Vernetzungschemikalien oder Vernetzungsbeschleuniger.

Um diesen Problemen zu begegnen, wurden im Stand der Technik bereits Verfahren zur Herstellung von Prophylaxeartikel mit reduziertem Allergiepotential vorgeschlagen.

Beispielsweise beschreibt die WO 2011/068394 A1 ein Verfahren, nach dem einem carboxylierten Nitrilbutadien eine Methacrylsäure und ZnO zugesetzt werden. Dadurch erlangt diese Mischung selbstvernetzende Eigenschaften, sodass auf Schwefelvernetzer und Beschleuniger verzichtet werden kann. Nach wie vor enthält diese Zusammensetzung aber das Schwermetall Zn, sodass ein gewisses Allergierestpotential verbleibt.

Ähnlich dazu beschreibt die US 2010/0152365 A1 die Verwendung eines carboxylierten Nitrilbutadien Copolymers zur Herstellung eines Handschuhs mittels Tauchverfahren. Wiederum wird ZnO zur ionischen Vernetzung eingesetzt.

Es ist weiter bekannt, die Oberfläche von Naturkautschukhandschuhen zu modifizieren, um dessen Allergiepotential zu reduzieren. So beschreibt beispielsweise die von der Anmelderin stammende US 2014/0096307 Al ein Verfahren zur Modifizierung der Oberfläche eines Elastomers mit ungesättigten Kohlenstoff-Kohlenstoff-Bindungen, die im Bereich der Oberfläche zumindest teilweise durch eine photochemische Reaktion mit zumindest einem Thiol gesättigt werden. Zur Sättigung können Feststoffpartikel verwendet werden, die kovalent an die Oberfläche des Handschuhs gebunden werden.

Ähnlich dazu beschreibt die ebenfalls auf die Anmelderin zurückgehende US 2014/0096308 Al u.a. die Anbindung von Zeolithpartikel an einen Naturkautschukhandschuh über Epoxidgruppen.

EP1762586A2 beschreibt ein Verfahren zur Herstellung von vernetztem Elastomer, indem eine Mischung aus Latex und einem Fotoinitiator als Starterkomponente hergestellt und mit UV- oder sichtbarem Licht bestrahlt wird, um die Vernetzung auszulösen. EP1209186A1 beschreibt ein nicht-adhäsives, carboxyliertes, getauchtes Latexprodukt, bei dem mindestens eine Oberfläche mit einem Carboxylgruppen-Blockierungsagens behandelt ist und dem Latex Aluminat oder Aluminiumhydroxidgel intern zugegeben wird.

WO00/11980 beschreibt medizinische Geräte, die nicht-allergen sind und für den Kontakt mit lebendem Gewebe von Menschen oder Tieren geeignet sind. Geräte bestehen aus einem nicht-allergenen synthetischen Polymer, das aus einer wässrigen Polymerdispersion gefertigt wird. Diese Polymere besitzen funktionelle Gruppen, die durch Reaktionsmittel vernetzbar sind, welche mit diesen funktionellen Gruppen reagieren können.

Brown (Rubber Chemistry and Technology (1963) 36 (4): 931-962) offenbart, dass Carboxylgruppen in Elastomermolekülen durch verschiedene Mechanismen zur Vernetzung beitragen, einschliesslich Wasserstoffbrückenbildung, Salzbildung, Reaktionen mit Epoxiden, Aminen, Carbodiimiden, Polyiminen und Polyisocyanaten. Diese Reaktionen können allein oder in Kombination mit anderen Vernetzungsprozessen wie Schwefel- oder Peroxidvulkanisation verwendet werden, besonders in Verbindung mit Zinkoxid bei Schwefelvulkanisationen. Carboxylgruppen können auch während der Vernetzungsreaktion entstehen.

Der vorliegenden Erfindung liegt die Aufgabe zugrunde, einen verbesserten Prophylaxeartikel zu schaffen.

Die Aufgabe wird durch das Verfahren gemäss Anspruch 1 gelöst.

Die Aufgabe der Erfindung wird auch durch die Verwendung eines mehrfach funktionellen Polymers mit einer Anzahl an Monomereinheiten zwischen 2 und 50 (einem Molekulargewicht zwischen 170 g/mol und 4000 g/mol) zur Einstellung des Moduls eines Prophylaxeartikels aus einem (carboxylierten) Dienelastomer gelöst, wobei das Polymer ausgewählt wird aus einer Gruppe bestehend aus mehrfachfunktionellen Epoxiden, mehrfachfunktionellen Silanen, mehrfachfunktionellen Siloxanen, mehrfachfunktionellen Thiolen, sowie Mischungen daraus.

Zudem wird die Aufgabe der Erfindung gelöst durch die Verwendung einer mehrfach funktionellen organischen Verbindung als Vernetzungsmittel zur Herstellung eines Prophylaxeartikels, wobei die mehrfach funktionelle organische Verbindung ein Molekulargewicht zwischen 170 g/mol und 4000 g/mol und zumindest zwei funktionellen Gruppen aufweist, und die unter basischer Katalyse Hydroxygruppen ausbildet, wobei das Vernetzungsmittel ausgewählt wird aus einer Gruppe bestehend aus mehrfachfunktionellen Epoxiden, mehrfachfunktionellen Silanen, mehrfachfunktionellen Siloxanen, mehrfachfunktionellen polymeren Thiolen, sowie Mischungen daraus.

Von Vorteil ist dabei, dass durch die chemische Reaktion des Vernetzungsmittels mit den Elastomermolekülen das Vernetzungsmittel besser in das vernetzte Elastomer eingebaut wird. Dadurch ist das Vernetzungsmittel nicht bzw. schwer aus dem Elastomer extrahierbar bzw. migriert nicht und nur sehr langsam aus dem Elastomer. Mit "sehr langsam" ist dabei gemeint, dass die Migrationszeit sehr viel größer ist, als die Anwendungszeit des Prophylaxeartikels. Es wird damit verhindert, dass das Vernetzungsmittel in Kontakt mit der menschlichen Haut gelangt, wodurch das Allergiepotential des Prophylaxeartikels deutlich gesenkt werden kann. Auch während der Lagerung der Prophylaxeartikel kann die Migration des Vernetzungsmittels aus dem Prophylaxeartikel verhindert bzw. deutlich verringert werden. Zudem können dadurch Leachingprozesse zur Entfernung nicht gebundener Prozesschemikalien verkürzt oder sogar eingespart werden. Das Vernetzungsmittel kann ein mehrfach funktionelles Monomer und/oder Polymer bzw. Mischungen daraus sein. Mit dem Verfahren kann ein Prophylaxeartikel hergestellt werden, der sehr gute mechanische Eigenschaften und eine hohe Alterungs- und Gammabeständigkeit aufweist. Auch konnte eine Beeinflussung der Filmbildung während des Herstellungsprozesses, insbesondere des Tauchprozesses, nicht nachgewiesen werden, sodass also keine weiteren Maßnahmen hierfür erforderlich sind. Ein weiterer Vorteil des Verfahrens ist darin zu sehen, dass keine Vorvernetzung des (carboxylierten) Dienlatex erforderlich ist, sodass kontinuierliche Mischverfahren eingesetzt und die Prozessabläufe beschleunigt werden können. Es ist mit dem Verfahren eine energieeffiziente, nachhaltige und produktionseffiziente Herstellung von hypoallergenen Prophylaxeartikeln, insbesondere Operations- und Untersuchungshandschuhen, möglich. Durch die Wasserlöslichkeit des Vernetzungsmittels wird bei dessen Einbringung in die Latexmischung kein bzw. nicht zwingend ein Emulgator benötigt. Die mehrfach funktionellen Monomeren und/oder Polymeren haben den Vorteil der einfacheren Handhabung, da das lösliche Monomer und/oder Polymer ohne vorhergehende Dispersion oder Emulsion in den Latex eingemischt werden kann. Die Einmischung in Form einer Emulsion ist aber möglich, insbesondere im Falle von öllöslichen Monomeren und/oder öllöslichen Polymeren. Zudem kann auch das Modul des Prophylaxeartikels besser eingestellt werden.

Nach einer bevorzugten Ausführungsvariante des Verfahrens kann vorgesehen sein, dass als Vernetzungsmittel ausschließlich das mehrfach funktionelle Monomer und/oder Polymer verwendet wird. Es können damit die voranstehend genannten Effekte weiter verbessert werden, wobei zusätzlich noch erreicht werden kann, dass durch den Verzicht von Schwermetallionen, wie beispielsweise Zn²⁺ aus ZnO, das Allergiepotential weiter verringert werden kann (Zink kann beispielsweise mit Carbonsäuren, wie z.B. Essigsäure, aus dem Elastomer extrahiert werden). Darüber hinaus kann keine Beeinflussung eines anderen Vernetzersystems stattfinden, wie dies teilweise im Stand der Technik berichtet wird.

Die Vernetzung der (carboxylierten) Dienlatexmoleküle kann thermisch durchgeführt werden. Somit kann die Vernetzung der Latexmoleküle bereits während des Trocknens des auf die Tauchform aufgetauchten Latexfilms erfolgen, wodurch eine Effizienzsteigerung des Verfahrens erzielbar ist.

Es ist weiter möglich, die Vernetzung der (carboxylierten) Dienlatexmoleküle photochemisch mittels UV-Licht durchzuführen. Es kann damit die Alterungsbeständigkeit des Elastomers verbessert werden. Auch hinsichtlich hochenergetischer Strahlung weisen die Elastomerprodukte eine verbesserte Stabilität auf. Dies ist insbesondere in Hinblick auf die Sterilisation der Medizinprodukte mit Gammastrahlung von Bedeutung. Zudem kann durch dieses Verfahren der Einsatz von Typ IV allergenen Substanzen ebenfalls einfacher vermieden werden.

Der pH-Wert des (carboxylierten) Dienlatex ist auf einen Wert von größer/gleich 9 eingestellt. Es konnte mit pH-Werten ab 9 eine deutliche Verbesserung der Reaktionskinetik beobachtet werden, wodurch die Vernetzung der Moleküle rascher erfolgen kann. Das Vernetzungsmittel wird ausgewählt aus einer Gruppe bestehend aus mehrfachfunktionellen Epoxiden, mehrfachfunktionellen Silanen, mehrfachfunktionellen Siloxanen, mehrfachfunktionellen polymeren Thiolen. Von Vorteil ist dabei, wenn diese (i) mehr als eine Epoxidfunktion für die Vernetzung der Kautschukketten haben. Vorzugsweise wiesen die mehrfachfunktionellen Epoxide eine Struktur auf, dass das Hydrolyseprodukt "pflegende" Eigenschaften aufweist, wie beispielsweise diglycidylterminiertes Polyethylenglykolderivat, Epoxy-Sorbitolderivat, Derivat eines Zuckeralkohols. Weiter können beispielsweise. Mono- und Polysaccharide mit Epoxy-Funktionalitäten eingesetzt werden.

Bei carboxylierten Dienlatices bietet die Vernetzung mit Epoxiden den Vorteil, dass kovalente Netzwerkstellen über die Carboxylgruppen gebildet werden und dadurch sehr hohe Reißfestigkeiten resultieren - die kovalente Vernetzung über die C=C Doppelbindungen der Butadieneinheiten bringt hingegen kaum eine Verbesserung in den Festigkeiten.

Weiterer Vorteil von Epoxiden ist die hohe Reaktivität mit Carboxylgruppen (man benötigt keinen zusätzlichen Beschleuniger oder Initiator), die zu einer effizienten Vernetzung während des Trocknungsschrittes führt.

Von Vorteil bei den mehrfachfunktionellen polymeren Thiolen ist, wenn diese (i) eine hohe Molmasse besitzen (Molmasse zwischen 200 g/mol und 4000 g/mol); (ii) eine hohe Mercaptoequivalentzahl aufweisen (zumindest 20 %, insbesondere zumindest 50%, der Monomereinheiten sollten SH-Gruppen tragen); (iii) über einfache Synthesestrategien zugänglich sind. Auf Grund des hohen Molekulargewichts können auch Geruchsproblem besser gehandhabt werden.

Von Vorteil bei den mehrfachfunktionellen Silane und Siloxane ist, wenn diese (i) mehr als eine reaktive Gruppe tragen (z.B. CoatOSil MP200 führt zu höheren Reißfestigkeiten als 3-Glycidoxypropyltrimethoxysilan). Es ist dabei weiter von Vorteil, dass diese auch bei hohen Molekulargewichten (z.B. bis 4000 g/mol) noch flüssig sind und dadurch leichter in die Latexmischung eingebracht werden können. Mit den Siloxanen kann darüber hinaus durch ihren flexiblen back bone eine zu große Erhöhung des Moduls vermieden werden.

Zur besseren Einstellung des Moduls des Prophylaxeartikels kann vorgesehen sein, dass ein organisches Monomer und/oder Polymer eingesetzt wird, das ein Molekulargewicht zwischen 170 g/mol und 4000 g/mol aufweist. Es kann damit ein besserer Tragekomfort für den Benutzer des Prophylaxeartikels erreicht werden.

Wie bereits ausgeführt, besteht auch die Möglichkeit, das Vernetzungsmittel dem (carboxylierten) Dienlatex als Emulsion zuzusetzen. Durch die feine Verteilung des Vernetzungsmittels in der Emulsion kann ein homogenerer Prophylaxeartikel einfacher erreicht werden.

Das Vernetzungsmittel ist dem (carboxylierte) Dienlatex in einem Anteil von 1 phr bis 10 phr, bezogen auf die Gesamtzusammensetzung des (carboxylierten) Dienlatex, zugesetzt Es kann damit besser vermieden werden, dass der 50 % Modul zu hoch wird, wodurch die Tragbarkeit des Prophylaxeartikels leiden würde. Gleichzeitig werden aber mit einer Konzentration des Vernetzungsmittels aus diesem Bereich gute mechanische weitere Eigenschaften des Elastomers, wie beispielsweise die Reißfestigkeit oder die maximale Dehnbarkeit, erhalten. Bevorzugt wird auch zumindest ein Fotoinitiator in einem Anteil von 0,5 phr bis 5 phr zugesetzt. Als Fotoinitiator können insbesondere α-Hydroxyalkylphenone, α-Aminoalkylphenone, Acylphosphinoxide, Benzoinether, Benzilketale, α-Dialkoxyacetophenone eingesetzt werden.

Zum besseren Verständnis der Erfindung wird diese anhand der nachfolgenden Figuren näher erläutert.

Es zeigen:
- Fig. 1: den Quellgrad von vernetzten XNBR-Latexfilmen über die Vernetzungszeit bei unterschiedlichen DEPEG-500 Konzentrationen;
- Fig. 2: den Quellgrad von vernetzten XNBR-Latexfilmen über die Vernetzungszeit bei unterschiedlichen GE-100-Konzentrationen;
- Fig. 3: den Quellgrad von vernetzten XNBR-Latexfilmen über die Vernetzungszeit bei unterschiedlichen SPE-Konzentrationen;
- Fig. 4: die Moduln (50% Dehnung) von vernetzten XNBR-Latexfilmen bei unterschiedlichen DEPEG-500-Konzentrationen;
- Fig. 5: die Moduln (50% Dehnung) von vernetzten XNBR-Latexfilmen (nicht steril / nicht gealtert) bei DEPEG-Typen mit unterschiedlichem Molekulargewicht;
- Fig. 6: die Moduln (50% Dehnung) von vernetzten XNBR-Latexfilmen (nicht steril / gealtert bei 70°C für 7 Tage) bei DEPEG-Typen mit unterschiedlichem Molekulargewicht;
- Fig. 7: die Reißfestigkeiten von UV vernetzten NR-Latexfilmen (nicht steril, nicht gealtert) bei unterschiedlichen Poly(mercaptopropylmethyl)siloxan-Konzentrationen (Synthesezeit: 3, 6 und 9h);
- Fig. 8: die Bruchdehnungen von UV vernetzten NR-Latexfilmen (nicht steril, nicht gealtert) bei unterschiedlichen Poly(mercaptopropylmethyl)siloxan-Konzentrationen (Synthesezeit: 3, 6 und 9h);
- Fig. 9: die Moduln (50% Dehnung) von UV vernetzten NR-Latexfilmen (nicht steril, nicht gealtert) bei unterschiedlichen Poly(mercaptopropylmethyl)siloxan-Konzentrationen (Synthesezeit: 3, 6 und 9h);
- Fig. 10: die Reißfestigkeiten von UV vernetzten NR-Latexfilmen (nicht steril, nicht gealtert) bei unterschiedlichen Poly(mercaptopropylmethyl)siloxan-Konzentrationen (Monomerkonzentration: 9 und 18% (w/v));
- Fig. 11: die Bruchdehnungen von UV vernetzten NR-Latexfilmen (nicht steril, nicht gealtert) bei unterschiedlichen Poly(mercaptopropylmethyl)siloxan-Konzentrationen (Mono-merkonzentration: 9 und 18% (w/v));
- Fig. 12: die Moduln (50% Dehnung) von UV vernetzten NR-Latexfilmen (nicht steril, nicht gealtert) bei unterschiedlichen Poly(mercaptopropylmethyl)siloxan-Konzentrationen (Monomerkonzentration: 9 und 18% (w/v)).

Sämtliche in der Beschreibung zitierten Normen beziehen sich auf die zum Anmeldezeitpunkt gegenständlicher Patentanmeldung gültige Fassung, sofern nichts anderes angegeben ist.

Die Erfindung bezieht sich auf ein Verfahren zum Herstellen eines Prophylaxeartikels.

Der Prophylaxeartikel ist bevorzugt ein Handschuh, insbesondere ein chirurgischer Handschuh (Operationshandschuh) oder ein Untersuchungshandschuh. Der Prophylaxeartikel kann aber beispielsweise auch ein Fingerling, ein Katheter, ein Kondom, ein (medizinischer) Ballon, ein Sauger, etc., sein. Generell ist der Prophylaxeartikel bevorzugt ein Tauchartikel, also ein Produkt, das mittels eines Tauchverfahrens hergestellt wird.

Im Folgenden wird nur mehr auf die Ausbildung des Prophylaxeartikels als Handschuh eingegangen. Die Ausführungen dazu können aber auch auf andere Elastomerartikel, insbesondere Tauchartikel die nach einem Tauchverfahren hergestellte werden, übertragen werden.

Der Handschuh umfasst ein Dienelastomer (Dienkautschuk), insbesondere ein carboxyliertes Dienelastomer, bzw. besteht aus diesem.

Das Elastomer der Elastomerschicht kann sowohl auf einem Natur- als auch auf einem Syntheselatex basieren. Diese können ausgewählt sein aus einer Gruppe umfassend oder bestehend aus Naturkautschuk (NR), Polyisopren-Latex (IR), Nitrilbutadienkautschuk-Latex (NBR), carboxylierter Nitrilbutadienkautschuk-Latex (XNBR), carboxylierter Butadienlatex (XBR), Chloropren-Latex (CR), Styrol-Butadien Latex (SBR), carboxylierte Latices hergestellt aus Polymer-Blends, sowie Mischungen daraus.

Insbesondere wird ein carboxylierter Nitrilbutadienkautschuk-Latex oder ein Polyisopren-Latex oder ein Naturkautschuk zur Herstellung der Elastomerschicht verwendet. Der Nitrilbutadienkautschuk-Latex weist bevorzugt einen Anteil an Acrylnitril zwischen 15 Gew.-% und 40 Gew.-%, insbesondere zwischen 20 Gew.-% und 35 Gew.-%, auf.

Der Prophylaxeartikel bzw. der Elastomerhandschuh wird bevorzugt nach einem Tauchverfahren hergestellt. Derartige Tauchverfahren sind aus dem Stand der Technik prinzipiell bekannt, sodass zu Einzelheiten dazu auf den einschlägigen Stand der Technik verwiesen sei.

Im Wesentlichen wird bei diesem Verfahren eine Tauchform (üblicherweise werden in der seriellen Fertigung mehrere Tauchformen verwendet) in ein Tauchbad eingetaucht. Die Tauchform weist dabei die Form des fertigen Produktes auf, also beispielsweise die Form einer Hand.

Im Tauchbad ist der jeweilige Elastomerlatex vorgelegt, der auf die Tauchform aufgetaucht werden soll.

Prinzipiell kann aber auch jede andere geeignete Form in den in dieser Beschreibung beschriebenen Verfahren verwendet werden, insbesondere wenn die Elastomerschicht nicht nach einem Tauchverfahren hergestellt wird. Die Elastomerschicht kann beispielsweise auch durch aufstreichen oder aufsprühen des Elastomerlatex auf eine Form hergestellt werden. Ebenso sind auch andere geeignete Verfahren der Aufbringung des Latex auf eine Form anwendbar.

Der Begriff Elastomerlatex wird in dieser Beschreibung dem üblichen Gebrauch in der Fachsprache entsprechend verwendet. Dementsprechend ist ein Elastomerlatex eine Dispersion aus unvernetzten oder vorvernetzten oder vernetzungsfähigen Polymermolekülen zur Herstellung eines Elastomers. Es können im Rahmen der Erfindung also auch vorvernetzte Elastomerlatices verarbeitet werden, wobei die Vorvernetzung insbesondere mittels dem in dieser Beschreibung genannten Vernetzungsmittel erfolgen kann, das ein mehrfach funktionelles Monomer und/oder Polymer ist, das dem (carboxylierten) Dienlatex zugesetzt und in diesem gelöst oder in diesem emulgiert bzw. dispergiert wird.

Es ist aber weiter möglich, dass der Elastomerlatex erst nach dem Aufbringen auf die Form, also der aufgebrachte Elastomerlatex, vernetzt wird.

Eine übliche Prozessroute eines Koagulations-Tauchverfahrens kann beispielsweise folgende Verfahrensschritte umfassen:
- waschen der Tauchform und entfetten mit einem organischen Lösungsmittel;
- vorwärmen der Tauchform;
- tauchen der Tauchform in ein erstes Tauchbad mit einem Koagulanten;
- Antrocknen des ersten aufgetauchten Schicht;
- tauchen der Tauchform in ein weiteres Tauchbad zur Ausbildung der Elastomerschicht;
- trocknen/vulkanisieren (vernetzen);
- abziehen des Tauchartikels von der Form.

Wie im Nachfolgenden noch näher ausgeführt wird, kann anstelle der thermischen Vernetzung der Elastomermoleküle auch eine photochemische Vernetzung mittels UV-Licht, gegebenenfalls nach Zusatz eines Fotoinitiators, durchgeführt werden. Der Fotoinitiator kann eine handelsüblicher Fotoinitiator sein und in üblichen Konzentrationen zugesetzt werden. Es sei hierzu auf die eingangs genannten US 2014/0096307 A1 und US 2014/0096308 A1 verwiesen, die in dem Umfang der Fotoinitiatoren und deren Konzentrationen im Latex zu gegenständlicher Beschreibung gehören.

Für den Fall, dass der Elastomerhandschuh mehrschichtig ausgebildet wird, können weitere Schichten aus dem ersten Elastomerlatex oder aus einem anderen Elastomerlatex oder einem anderen Polymer aufgetaucht oder generell aufgebracht werden. Beispielsweise kann als letzte Schicht eine Polymerschicht aufgetaucht werden, die nach dem Abziehen des Handschuhs von der Tauchform durch das dabei erfolgende Wenden des Handschuhs an die Innenseite des Handschuhs gelangt. Derartige Polymerschichten können beispielsweise als Gleitschichten ausgebildet sein, um die Anziehbarkeit des Elastomerhandschuhs zu verbessern.

Der Elastomerhandschuh kann also ein- oder mehrschichtig ausgebildet sein, wobei die einzelnen Schichten aus zueinander unterschiedlichen Werkstoffen oder aus den gleichen Werkstoffen bestehen können. Es ist auch möglich, dass zwei oder mehr Schichten des Elastomerhandschuhs aus dem gleichen Werkstoff und eine oder mehr Schichten aus einem dazu unterschiedlichen Werkstoff bestehen.

Da all dies an sich bekannt ist, soll nicht weiter darauf eingegangen werden.

Unter Werkstoffen werden dabei in dieser Beschreibung Elastomere und Polymere verstanden, wobei aber der Elastomerhandschuh zumindest eine Schicht aus einem Elastomer aufweist.

Die Begriffe Vulkanisation und Vernetzung werden in dieser Beschreibung synonym verwendet.

Zur Vernetzung des (carboxylierten) Dienelastomer-Latex wird diesem, d.h. insbesondere dem Tauchbad zur Herstellung der zumindest einen Schicht aus dem (carboxylierten) Dienelastomers, ein Vernetzungsmittel zugesetzt. Daneben kann der Dienelastomer-Latex bzw. das Tauchbad zumindest ein weiteres Additiv, wie beispielsweise zumindest einen Emulgator, zumindest ein Alterungsschutzmittel, zumindest einen Farbstoff, zumindest ein Antiozonat, aufweisen, wie sie an sich für die Herstellung von Tauchartikeln bekannt sind. Der Gesamtanteil an diesen Additiven kann zwischen 0,1 phr und 10 phr, bezogen auf die Gesamtzusammensetzung des Dienelastomer-Latex bzw. des Tauchbads, betragen.

Dem (carboxylierten) Dienelastomer-Latex wird ein Vernetzungsmittel auf Monomerer und/oder polymerer Basis zugesetzt und in dem (carboxylierten) Dienelastomer-Latex aufgelöst. Die Konzentration an Vernetzungsmittel ist zwischen 1 phr und 10 phr, insbesondere kann zwischen 1 phr und 7,5 phr, betragen.

In der bevorzugten Ausführungsvariante des Verfahrens werden keine weiteren Vernetzungsmittel verwendet, d.h. dass als Vernetzungsmittel ausschließlich das in dem (carboxylierten) Dienelastomer-Latex lösliche Monomer und/oder Polymer verwendet wird. Es kann jedoch, wie voranstehend bereits ausgeführt, zumindest ein Fotoinitiator zugesetzt werden.

Der Begriff "Polymer" im Sinne dieser Beschreibung umfasst dabei generell Moleküle ab zwei Monomereinheiten, also Moleküle ab Dimeren. Die mehrfachfunktionellen Monomere und/oder Polymere werden vorzugsweise ausgewählt aus einer Gruppe umfassend oder bestehend aus mehrfachfunktionelle(n) Epoxide(n), mehrfachfunktionelle(n) Silane(n), mehrfachfunktionelle(n) Siloxane(n), mehrfachfunktionelle(n) Thiole(n), sowie Mischungen daraus.

Beispiele dafür sind kurzkettige: Sorbitol polyglycidyl ether, Glycerol glycidyl ether, 1,6-Hexandioldiglycidyl ether, Resorcinol diglycidyl ether, 1,4-Cyclohexandimethanol diglycidyl ether, Diglycidyl 1,2-cyclohexandicarboxylat, langkettige: Diepoxy-terminiertes Polyethylenglykol, Diepoxy-terminiertes Polypropylenglykol, Polyglycidylmethacrylat (Homopolymere und Copolymere mit Ethylenglykoleinheiten, Ethyleneinheiten, etc.), Polyglycerinpolyglycidether, Polyglycidoxypropyltrimethoxysilan.

Kurzkettige Verbindungen sind monomere mehrfachfunktionelle Verbindungen, insbesondere derartige Verbindungen mit einem Molekulargewicht von zumindest 170 g/mol. Langkettige Verbindungen weisen zumindest zwei oder mehrere Wiederholungseinheiten auf (Dimere und größer).

Generell umfasst im Rahmen der Erfindung der Begriff "Polymer" auch Oligomere.

Gemäß einer anderen Ausführungsvariante des Verfahrens kann vorgesehen sein, dass als mercaptofunktionelles Siloxanpolymer ein mercaptofunktionelles Siloxanhomopolymer oder ein Copolymer des mercaptofunktionellen Siloxanhomopolymers mit einem Acrylsiloxan verwendet wird. Insbesondere wird ein mercaptofunktionelles Siloxanhomopolymer mit der Strukturformel 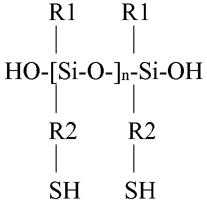 eingesetzt, wobei R1 für eine erste Einheit ausgewählt aus einer ersten Gruppe bestehend aus -CH₃, -OH, -C₂H₅, -C₃H₇, aromatische Gruppen, R2 für eine zweite Einheit ausgewählt aus zweiten Gruppe bestehend aus -CH₂, C₂H₄, C₃H₆; -(CH₂)₁₁-, aromatische Gruppen, -CH₂-Aromat, steht, und/oder ein Acrylsiloxan mit der Strukturformel 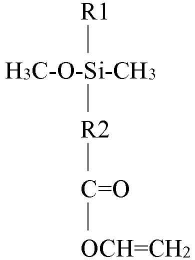 eingesetzt, wobei R1 für eine erste Einheit ausgewählt aus einer ersten Gruppe bestehend aus -OH, -CH₃, -C₂H₅, -C₃H₇, aromatische Gruppen, R2 für eine zweite Einheit ausgewählt aus zweiten Gruppe bestehend aus -CH₂, C₂H₄, C₃H₆; aromatische Gruppen, steht.

Als mercaptofunktionelles Siloxan kann auch das nachstehende Dimer eingesetzt werden.

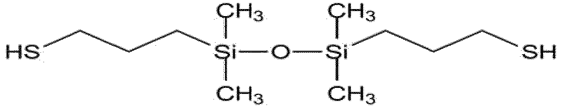

Nach einer besonders bevorzugten Ausführungsvariante des Verfahrens ist das mercaptofunktionelle Siloxanhomopolymer ausgewählt ist aus einer Gruppe bestehend aus Poly(mercaptomethylpropyl)siloxan, Poly(mercaptomethylpropyl)siloxan, Poly(mercaptomethylmethyl)siloxan, Poly(mercaptoethylmethyl)siloxan, Poly(mercaptomethylethyl)siloxan, Po-ly(mercaptopropylmethyl)siloxan, Poly(mercaptomethylbenzyl)siloxan, Po-ly(mercaptopropylbenzyl)siloxan, Poly(mercaptoethylbenzyl)siloxan und/oder das Copolymer des mercaptofunktionellen Siloxanhomopolymers mit einem Acrylsiloxan ausgewählt ist aus einer Gruppe bestehend aus Poly(mercaptomethylpropyl-co-acryloxymethylpropyl)siloxan, Poly(mercaptomethylmethyl-co-acryloxymethylpropyl)siloxan, Po-ly(mercaptomethylmethyl-co-acryloxypropylmethyl)siloxan, Po-ly(mercaptomethylmethyl-co-acryloxypropylethyl)siloxan, Po-ly(mercaptomethylmethyl-co-acryloxyethylpropyl)siloxan, Po-ly(mercaptomethylmethyl-co-acryloxymethylmethyl)siloxan, Po-ly(mercaptomethylmethyl-co-acryloxypropyl)siloxan, Poly(mercaptomethylmethyl-co-acryloxyethyl)siloxan, Poly(mercaptomethylmethyl-co-acryloxymethyl)siloxan, Poly(mercaptopropylmethyl-co-acryloxymethylpropyl)siloxan.

Der Anteil des mercaptofunktionellen Siloxanpolymers an dem Copolymer des mercaptofunktionellen Siloxanpolymers mit einem Acrylsiloxan kann ausgewählt sein aus einem Bereich von 20 Gew.-% bis 99 Gew.-%, insbesondere aus einem Bereich von 20 Gew.-% bis 80 Gew.-%.

Die Schichtdicke der Elastomerschicht kann zwischen 30 µm und 500 µm betragen.

Generell kann der (carboxylierte) Dienelastomer-Latex einen Feststoffgehalt an (carboxyliertem) Dienelastomer zwischen 10 drc (dry rubber content) und 60 drc aufweisen.

Der pH-Wert des (carboxylierten) Dienelastomer-Latex ist auf einen Wert von größer/gleich 9 eingestellt. Dazu kann beispielsweise eine wässerige KOH-Lösung (1Gew.-% bis 5 Gew.-%) verwendet werden. Generell können dazu geeignete basische Substanzen, wie Laugen, verwendet werden.

In einer bevorzugten Ausführungsvariante des Verfahrens erfolgt die Vernetzung der (carboxylierten) Dienelastomermoleküle thermisch, insbesondere während der Trocknung der (aufgetauchten) Schicht aus dem (carboxylierten) Dienelastomer-Latex. Die Temperatur kann dabei zwischen 90 °C und 140 °C betragen. Die Vernetzung kann während einer Zeitspanne zwischen 5 Minuten und 20 Minuten erfolgen.

Es kann ein Vernetzungsmittel eingesetzt werden, das ein Molekulargewicht zwischen 170 g/mol und 4000 g/mol, insbesondere zwischen 170 g/mol und 1700 g/mol (polymere, wasserlösliche Verbindungen gemäß DIN 55672-3:2007-08 (GPC)) oder über die Viskosität von flüssigen Polymeren gemäß DIN 51 562-1) aufweist. Beispielsweise kann Ethylenglykoldiglycidylether (Molekulargewicht 170 g/mol) oder Diethylenglykoldiglycidylether (Molekulargewicht 218 g/mol) verwendet werden. Es ist damit auch möglich, den (50%-)Modul des Elastomerhandschuhs auf einen gewünschten Wert einzustellen. Das Modul des Elastomerhandschuhs kann über die Kettenlänge des Vernetzungsmittels eingestellt werden.

Mit dem Verfahren kann ein Prophylaxeartikel, insbesondere Handschuh, hergestellt werden, umfassend eine Schicht aus einem (carboxylierten) Dienelastomer, wobei die (carboxylierten) Dienelastomermolekülketten des (carboxylierten) Dienelastomers kovalent über organische Moleküle vernetzt sind.

Die nach dem Verfahren hergestellten Elastomerhandschuhe weisen eine gute Hautverträglichkeit auf. Anhand von durchgeführten Untersuchungen konnte kein Hautreizungs- und kein Sensibilisierungspotential festgestellt werden.

Im Zuge der Erprobung des Vernetzungsverfahrens wurden u.a. folgende Experimente durchgeführt. Bei diesen handelt es sich nur um ausgewählte Beispiele, da die Wiedergabe sämtlicher Experimente den Rahmen dieser Beschreibung sprengen würde.

Im Folgenden sind die Versuchsergebnisse für die Ausführung des Verfahrens mit mehrfachfunktionellen Monomeren und/oder Polymeren als Vernetzungsmittel wiedergegeben. In Tabelle 1 sind die hierfür verwendeten Edukte zusammengefasst.

**Tabelle 1: verwendete Materialien**

| *Name* | *Funktion* | *Beschreibung* |
|---|---|---|
| Nipol LX556 | Latex | 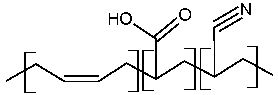 |
| ZEON Corporation (JPN) | | |
| BST8502N | | XNBR |
| PolyLac 582N | | Trockenkautschukgehalt: 45,2% pH-Wert: 8 bis 8.8 |
| SPE, Epoxy-Sorbit | Polymeres Vernetzungsmittel | 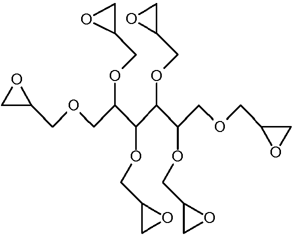 |
| CVC Thermo-set Specialities | | |
| | | Sorbitol polyglycidyl ether (ERISYS GE 60) |
| GE100 | Polymeres Vernetzungsmittel | 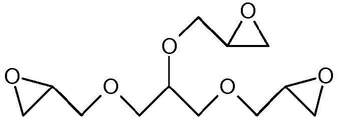 |
| Raschig | | |
| | | Glycerol glycidyl ether |
| DEPEG | Polymeres Vernetzungsmittel | 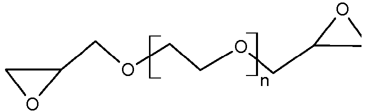 |
| Sigma-Aldrich (USA) | | |
| PolyScience (USA) | | |
| | | Diepoxy-terminiertes Polyethylenglykol |
| | | |
| | | DEPEG-200 Mn=200 |
| | | DEPEG-500 Mn=500 |
| | | DEPEG-1000 Mn=1000 |

### Herstellung der Latexmischungen, Tauchung und Vernetzung

Das wasserlösliche Vernetzungsmittel wurde in unterschiedlichen Konzentrationen (0,5 bis 1,5phr) der Latexmischung (pH = 10, ~25 drc.) zugegeben. Anschließend wurde die Mischung mit einem Alterungsschutzmittel (0,5 phr bis 2 phr Ralox) versetzt und etwa 15min bei Raumtemperatur gerührt. Anschließend wurden die Filme mittels dem voranstehend beschriebenen Koagulationstauchverfahren hergestellt und die Filme bei 100°C für 15min getrocknet. Es wurde keine Vorvernetzung bzw. Latexreifung benötigt. Die Vernetzung fand während der Trocknung der Filme bei 100°C statt.

Die Latexmischung kann während des Tauchprozesses leicht mit Hilfe eines Magnetrührers gerührt werden. Dies trifft generell auf das in dieser Beschreibung beschriebene Verfahren zu.

Nachfolgende Reaktionen liegen der thermischen Vernetzung mit Monomeren und/oder polymeren Epoxidvernetzern zu Grunde. Von Vorteil ist im Vorfeld die Einstellung des pH-Werts der Latexmischung, beispielsweise mit 1 Gew.-%iger KOH auf pH=10, da die Reaktion bei höheren pH-Werten katalysiert wird.

Reaktion eines carboxylierten Elastomers mit einem Epoxid

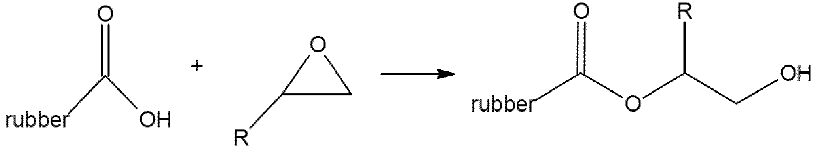

Sauer und basisch katalysierte Ringöffnung von Epoxiden.

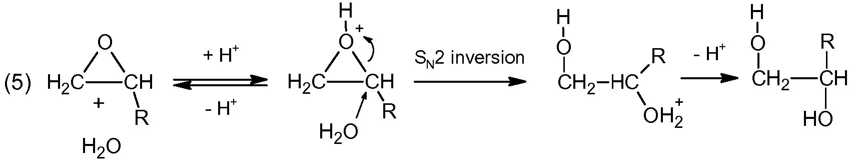

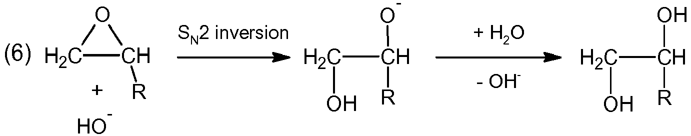

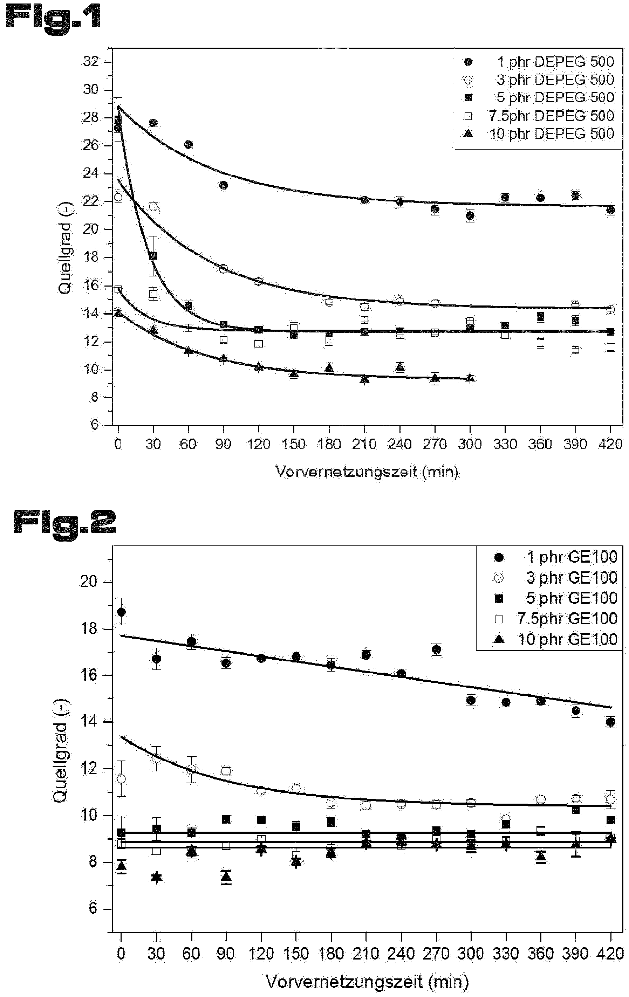

Die erfolgreiche Vernetzung von XNBR-Latex bei Zugabe von ausgewählten wasserlöslichen polymeren Vernetzungsmittels wurde mittels Gleichgewichtsquellung in Chloroform (bestimmt nach: (1) Macromolecules 2008, 41, 4717-4729, (2) J. Appl. Polym. Sci. 129(5), 2735-2743 bzw. (3) Zaborski, M.; Kosmalska, A.; Gulinski, J. Kautsch. Gummi Kunstst. 2005, 58, 354) nachgewiesen. Die Ergebnisse sind in den Fig. 1 bis 3 dargestellt. Dabei sind auf den Abszissen die Vernetzungszeit in Minuten und auf den Ordinaten der Quellgrad dargestellt. Die Vernetzungsdichte steigt hierbei mit zunehmender Vernetzungszeit und Vernetzungsmittelkonzentration wobei die Reaktivität der Vernetzungsmittel von DEPEG-500 < SPE < GE100 zunimmt.

Neben der Gleichgewichtsquellung wurde die Vernetzung von XNBR-Latex bei Zugabe von ausgewählten wasserlöslichen polymeren Vernetzungsmittel auch mittels Zugprüfung nachgewiesen.

Bei Einsatz von DEPEG-500 können ab einer Konzentration von 5 phr mechanische Festigkeiten im Bereich von 22 ± 2 MPa beobachtet werden. Bei geringeren Konzentrationen (0,5 bis 3 phr) wird eine geringe Vernetzungsdichte erzielt und die Reißfestigkeiten liegen unter 10 MPa. Eine Erhöhung der Vernetzerkonzentration auf 7,5 phr bringt eine weitere Erhöhung der Festigkeiten auf bis zu 35 ± 2 MPa. Bevorzugt wird daher eine Konzentration von 5 phr bis 7,5 phr.

Sehr gute mechanische Festigkeiten und Alterungs- bzw. Gamma-Beständigkeiten wurden auch mit DEPEG-200 in einem Konzentrationsbereich zwischen 3 phr und 7,5 phr beobachtet (nicht steril/ nicht gealtert: 26 MPa - 40 MPa; nicht steril/gealtert: 37 MPa -26 MPa; steril/nicht gealtert: 28 MPa -24MPa; steril/ gealtert: 25 MPa -35MPa).

Da ähnliche Ergebnisse auch mit anderen mehrfachfunktionellen Monomeren bzw. polymeren Vernetzungsmitteln erzielt wurden, wird generell eine Konzentration von 1 phr bis 7,5 phr mehrfachfunktionelle Monomeren und/oder polymere Vernetzungsmittel im Latex bevorzugt.

Weiter wird eine ausgezeichnete Heißluftalterungs- (7 Tage Lagerung bei 70°C) und GammaBeständigkeit (25 kGy) beobachtet.

Generell sei angemerkt, dass im Zuge der Tests des Prophylaxeartikels die Sterilisation durch Gamma-Strahlung mit einer Co-60-Quelle und einer Bestrahlungsdosis von 25kGy erfolgen kann. Die Alterung kann generell durch Heißluftalterung bei 70°C im Umlufttrockenofen über 7Tage erfolgen.

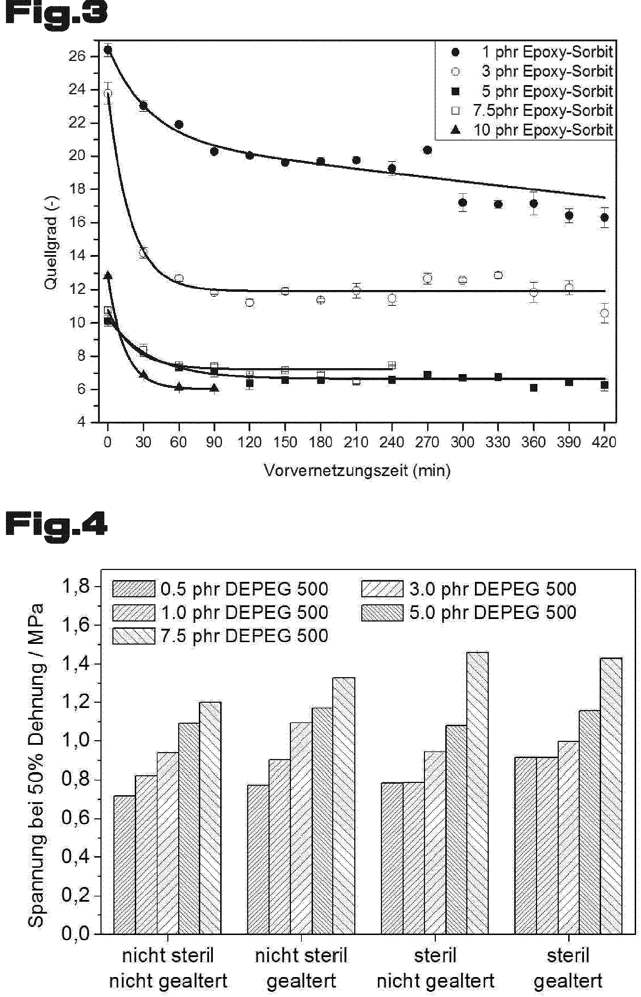

Zusätzlich liegt der Spannungswert bei 50% Dehnung auch bei hohen Reißfestigkeiten im Bereich von 1,2 bis 1,4 MPa und wird insbesondere bei Einsatz von 5 phr Vernetzungsmittel auch nach Heißluftalterung und Gammasterilisation kaum erhöht. Dies ist vor allem für die Herstellung von Operationshandschuhen von Vorteil, da ein geringer Spannungswert bei 50 % Dehnung ein Kriterium für einen angenehmen Tragekomfort darstellt. Die Ergebnisse der Messung der 50 % Dehnung sind in Fig. 4 dargestellt. Die Balken sind darin in Fünfergruppen angerordnet, wobei innerhalb jeder Fünfergruppe die Balken für eine Konzentration an DEPEG-500 von links nach rechts von 0,5 phr, 1,0 phr, 3,0 phr, 5,0 phr und 7,5 phr stehen. Die Fünfergruppen selbst stehen von links nach rechts für nicht sterile und nicht gealterte, nicht sterile und gealterte, sterile und nicht gealterte sowie sterile und gealterte Proben. Auf der Ordinate sind die 50 % Moduln in MPa angegeben.

Analog zur Vernetzung mit DEPEG-500 wurden auch bei Einsatz von SPE (Epoxy-Sorbit) sehr gute mechanische Eigenschaften (auch nach Gamma-Sterilisation) bei höheren Konzentrationen (7,5 phr) nachgewiesen. Bei einer Konzentration von 7,5 phr SPE wurden Werte für die mechanischen Eigenschaften zwischen 12 MPa und 32 MPa gemessen (nicht steril/ nicht gealtert: 30 MPa - 32 MPa; nicht steril/gealtert: 12 MPa -14 MPa; steril/nicht gealtert: 30 MPa -32MPa; steril/ gealtert: 13 MPa -15MPa). Bei einer Konzentration von DEPEG-500 zwischen 0,5 phr und 1,0 phr wurden hingegen nur Werte von maximal ca. 5 MPa gemessen. DEPEG-500 wird deshalb bevorzugt in einer Menge von 5 phr bis 7,5 phr eingesetzt.

Bei Verwendung von SPE als wasserlösliches polymeres Vernetzungsmittel wird zusätzlich

eine ausgeprägte Erhöhung des Spannungswertes bei 50 % Dehnung beobachtet, was nachteilig für den Tragekomfort des Elastomerhandschuhs ist. Bei 7,5 phr SPE werden Werte im Bereich von 1,6 bis 1,8 MPa erhalten. SPE wird daher vorzugsweise in einer Konzentration von 0,5 phr bis 5 phr eingesetzt.

Bei Einsatz von GE100 als Vernetzungsmittel werden bereits bei geringen Konzentrationen (1 und 3phr) sehr gute mechanische Festigkeiten erzielt, die im Bereich von 20 bis 27 MPa liegen. Mit höheren Vernetzungsmittelkonzentrationen (7,5 phr) wird eine weitere Erhöhung der Reißfestigkeiten beobachtet (37 ± 2MPa). Bei einer Konzentration von 5 phr werden Werte zwischen 22 MPa und 40 MPa erhalten (nicht steril/ nicht gealtert: 35 MPa - 40 MPa; nicht steril/gealtert: 32 MPa -35 MPa; steril/nicht gealtert: 36 MPa -38MPa; steril/ gealtert: 22 MPa -23MPa). Die vernetzten XNBR-Latexfilme zeichnen sich durch eine sehr gute Gamma-Beständigkeit aus.

Zusammenfassend kann aus den Ergebnissen geschlossen werden, dass hohe Reißfestigkeiten (30 ± 2MPa) und Gamma-Beständigkeiten (nach Gammasterilisation: 30 ± 2MPa) mit allen drei untersuchten Vernetzungsmitteln erzielt worden sind. Hinsichtlich Beständigkeit gegenüber einer Heißluftalterung oder einem geringen Modul bei 50 % Dehnung weist DEPEG-500 klare Vorteile gegenüber GE-100 und SPE auf.

Basierend auf diesen Ergebnissen wurde in weiteren Untersuchungen der Modulwert der vernetzten XNBR-Latexfilme durch das Molekulargewicht des epoxid-terminierten Polyethylenglykolderivats (DEPEG) gezielt eingestellt. Mit niedrigerem Molekulargewicht wird einerseits eine sehr hohe Festigkeit (bis zu 40MPa) erreicht, während der Modul ansteigt. Dies ist vor allem für die Herstellung von Untersuchungshandschuhen interessant, wo hohe Festigkeiten im Vordergrund stehen und der Modul (auf Grund der Schichtdicke) nur eine untergeordnete Rolle spielt. Bei XNBR-Filmen, die mit DEPEG-500 (mittleres Molekulargewicht) vernetzt worden sind, erhält man zwar etwas geringere Festigkeiten, aber die Modulwerte sind wesentlich geringer. Diese Variante eignet sich mehr für die Herstellung von Operationshandschuhen, wo das Hauptaugenmerk auf einen niedrigen Modul liegt.

Liegt das Molekulargewicht des Vernetzungsmittels jedoch im Bereich von 1.000 g/mol, kann zwar der 50 % Modulwert unter 1MPa gebracht werden, aber die entsprechenden Reißfestigkeiten liegen auch unter 15MPa. Die Ergebnisse zeigen daher, dass über die Kettenlänge des Vernetzungsmittels eine Balance zwischen Reißfestigkeit und Modul eingestellt werden kann. Es werden daher die voranstehend angeführten Kettenlängen der polymeren Vernetzungsmittel bevorzugt.

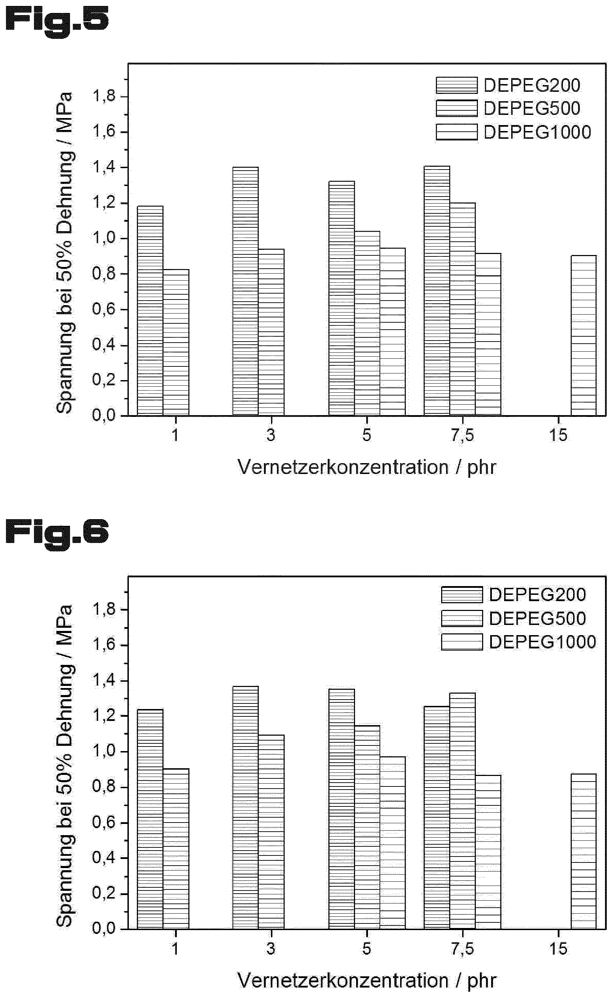

Die Messergebnisse dieser Untersuchung sind in den Fig. 5 und 6 dargestellt. Dabei sind auf den Abszissen die Konzentration an Vernetzungsmittel in phr und auf den Ordinaten die gemessenen Spannungen bei 50 % Dehnungen in MPa aufgetragen.

In weiteren Untersuchungen wurde PolyLac 582N als weitere alternative Latextype bei unterschiedlichen pH-Werten mit 5 phr DEPEG-200 vernetzt. Die Ergebnisse zeigen deutlich, dass eine erfolgreiche Vernetzung von PolyLac 582N gelingt.

Im Folgenden werden ausgewählte Beispiele zur photochemischen Vernetzung von Elastomerlatices wiedergegeben. In Tabelle 2 sind die hierfür verwendeten Edukte zusammengefasst.

**Tabelle 2: verwendete Materialien**

| | | |
|---|---|---|
| *Name* | *Funktion* | *Beschreibung* |
| Naturkautschuk-Latex | Latex | NR |
| | | High ammonia, 60% drc |
| Isoprenkautschuk-Latex Kraton | Latex | 60% drc |
| 3-Mercaptopropyl-trimethoxysilan | Monomer für Synthese des polymeren Vernetzers | 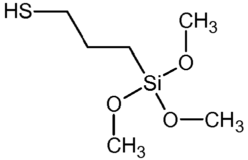 |
| ABCR | | |
| | | MPTMS |
| 3-Mercaptopropyl-methyldimethoxysilan | Monomer für Synthese des polymeren Vernetzers | 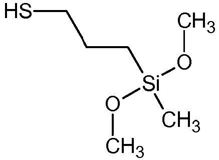 |
| ABCR | | |
| | | MPMDMS |
| 3-Mercaptomethyl-methyldimethoxysilan | Monomer für Synthese des polymeren Vernetzers | 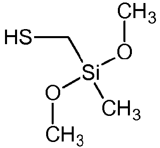 |
| ABCR | | |
| | | MMMDMS |
| 3-Acryloxypropyl-methyldimethoxysilan | Monomer für Synthese des | 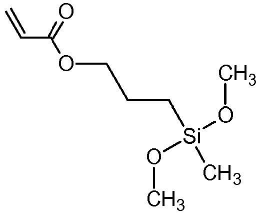 |
| ABCR | polymeren Vernetzers | |
| | | APMDMS |
| Lucirin TPO-L | Photoinitiator | 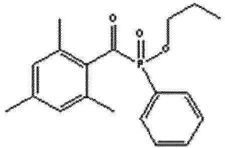 |
| BASF | | |
| Tween 20 | Emulgator | 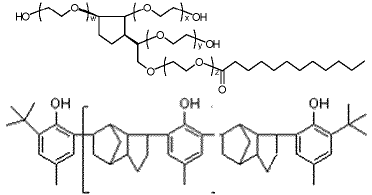 |
| Ionol LC | Antioxidant | |

Die Herstellung des polymeren Siloxanvernetzungsmittels kann wie folgt erfolgen.

0,1 M HCl (aq.) und Ethanol werden vorgelegt, auf 50 °C erhitzt und mit einem kontinuierlichen N₂-Strom gespült. Anschließend werden die entsprechenden Siloxanmonomere (siehe Tabelle 2) in ausgewählten Konzentrationen von 5 % (w/v) und 40 % (w/v) hinzugefügt. Nach 3 bis 9 Stunden bei 50°C wird die Reaktion durch Abkühlung gestoppt und das ölige Produkt mit deionisiertem Wasser gewaschen und mit Chloroform extrahiert. Das Lösungsmittel wird im Abschluss unter Vakuum abgezogen und das Produkt unter N₂-Atmosphäre gelagert.

Nachfolgend sind die Reaktionsschemata der synthetisierten Homo- und Copolymere aufgelistet.

### Synthese von Poly(mercaptopropyl)siloxan:

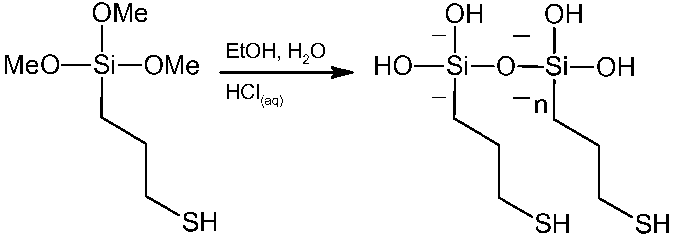

### Synthese von Poly(mercaptomethylmethyl)siloxan:

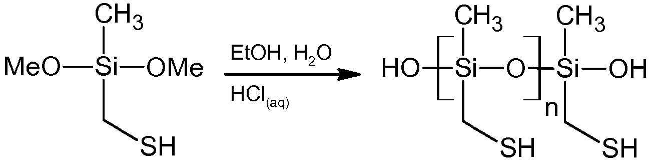

### Synthese von Poly(mercaptopropylmethyl)siloxan

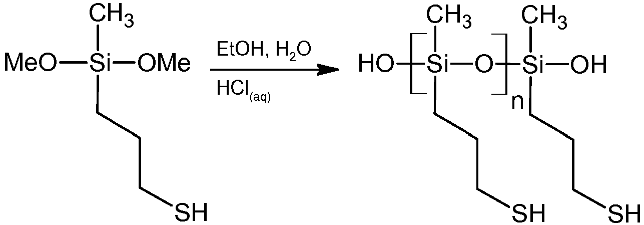

### Synthese von Poly(mercaptopropylmethyl-co-acryloxypropylmethyl)siloxan

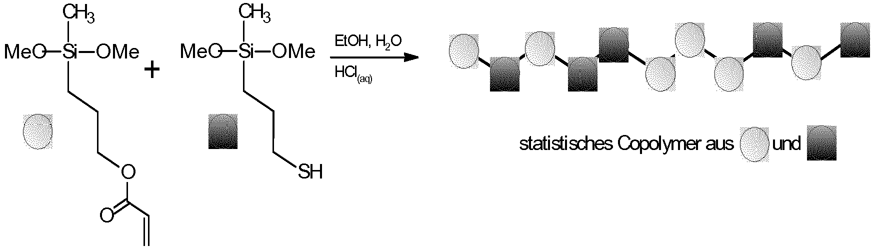

Die Molekulargewichtsverteilung der Siloxane wurde mittels Gelpermeationschromatographie (Universalkalibration mit Polystyrolstandards) bestimmt. Folgende Ergebnisse wurden erhalten:
- Poly(mercaptopropylmethyl)siloxan (3 Stunden - Reaktionszeit)
   Molekulargewicht: 200 g/mol - 700 g/mol (2 bis 5 Einheiten)
- Poly(mercaptopropylmethyl)siloxan (9 Stunden - Reaktionszeit)
   Molekulargewicht: 200 - 1.400 g/mol (2 bis 10 Einheiten)
- Poly(mercaptopropylmethyl-co-acryloxypropylmethyl)siloxan (3 Stunden Reaktionszeit)
   Molekulargewicht: 200 - 1.300 g/mol

### Herstellung der Latexfilme und UV-Vernetzung mit polymeren Siloxanvernetzungsmitteln

Die synthetisierten polymeren Vernetzungsmittel werden in unterschiedlichen Konzentrationen (1 bis 4 phr) mit Lucirin TPO-L (1phr) in deionisiertem Wasser mit Tween 20 (0,1 phr) emulgiert und anschließend dem NR-Latex (40 drc.) zugegeben. Die Latexmischung wird mit einem Alterungsschutzmittel (0,5 phr Ionol LC) versetzt und zwei Stunden bei Raumtemperatur gerührt. Anschließend werden die Filme mittels nachfolgendem Koagulationstauchverfahren hergestellt:
- Waschen der Keramikformen und Entfetten mit Aceton
- Vorwärmen der Keramikformen für mindestens 10 min im Trockenschrank bei 120°C
- Tauchen der Form für 30 s in das Koagulationsbad bei 70°C
- Trocknen der Form für mindestens 1 min im Trockenschrank bei 120°C
- Tauchen der Form in die NR-Latexmischung für 20s
- Trocknen für 20 min im Trockenschrank bei 120°C
- Abziehen des Films

Die UV-Vernetzung der NR-Latexfilme erfolgte im Zuge einer UV-Belichtung der getrockneten Filme (Post-Curing) mit einem UV-Strahler von Fusion UV Systems Inc. Die UV-Belichtung erfolgte unter Luft mit einem Ga-dotierten Hg-Strahler bei einer Lampenleistung von 60% und einer Bandgeschwindigkeit von 3,5 m/min. Bei dreimaligem Durchlauf entspricht die Strahlungsdosis 15,6 J/cm².

Es sei darauf hingewiesen, dass die angegebenen Parameter nicht beschränkend zu verstehen sind, sondern lediglich einen Weg aufzeigen, die Prophylaxeartikel beispielsweise im Labormaßstab herzustellen. In der großtechnischen Anwendung können geringfügig andere Parameter erforderlich sein, die jedoch anhand weniger Versuche aufgefunden werden können.

Nachfolgender Reaktionsmechanismus liegt der photochemischen Vernetzung mit polymeren Siloxanvernetzungsmitteln zu Grunde.
(1) Initiation: 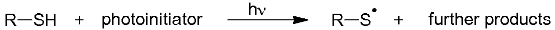
(2) Propagation: 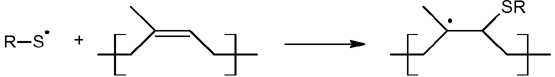 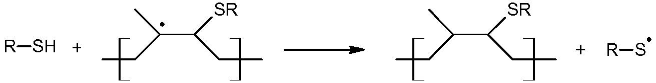
(3) Termination:

   R-S^{•} + R-S^{•} → R-S-S-R

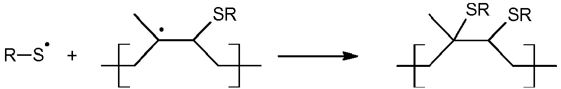

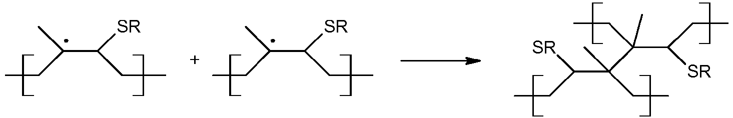

### Generell können folgende Paramter für die UV-Vernetzung verwendet werden:

IR-Latices - Parameter für UV-Vorvernetzung im Fallfilmreaktor: Strahlerleistung bei 800 W -1000 W (800 W ergibt einen mittleren Strahlungsfluss von ~ 500 mW/cm₂), zweifacher Belichtungsdurchgang, Fördergeschwindigkeit (Latexmischung) bei 1,1 l/min bis 1,5 l/min, Feststoffgehalt (Latex) bei 40 % drc., Fotoinitiatorkonzentration bei 0,5 phr bis 2phr, Thiolkonzentration bei 0,5 phr bis 2phr.

NR-Latices - Parameter für UV-Vorvernetzung im Fallfilmreaktor: Strahlerleistung bei 2000 W- 3500 W (3000 W ergibt einen mittleren Strahlungsfluss von ~ 1690 mW/cm²) zweifacher Belichtungsdurchgang, Fördergeschwindigkeit (Latexmischung) bei 1,1 l/min bis 1,5 l/min, Feststoffgehalt (Latex) bei 40 % drc., Fotoinitiatorkonzentration bei 0,5 phr bis 2phr, Thiolkonzentration bei 1 phr bis 5phr.

Generelle Parameter für UV-Nachvernetzung: Restfeuchtegehalt der Filme bevorzugt unter 20 %. Nachdosierung von 0,5 phr bis 5 phr Fotoinitiator und 1 phr bis 7,5 phr Thiol, Bestrahlungsdosis zwischen 1 J/cm² und 25 J/cm² (240 nm - 420 nm Wellenlängenbereich).

Die Belichtung erfolgt vorzugsweise unter Luft mit einem Ga-dotierten Hg-Strahler.

Die Struktur der polymeren Vernetzungsmittel wurden mittels FT-IR Spektroskopie und mittels Thermogravimetrie (TGA) bestimmt. In den FT-IR Spektren der mercaptofunktionellen Siloxanhomopolymere Poly(mercaptopropyl)siloxan, Poly(mercaptomethylpropyl)siloxan und Poly(mercaptomethylmethyl)siloxan kann eine signifikante Verringerung der Si-O-CH₃ Bande bei ca. 2830 cm⁻¹ sowie die Bildung von OH-Gruppen (ca. 3370 cm-1) beobachtet werden, die auf eine erfolgreiche Kondensationsreaktion der Siloxanmonore (Alkoxysilanmonomere) rückschließen lassen. Weiter weist die Verbreiterung der Si-O-Bande bei ca. 1060 cm⁻¹ auf die Bildung einer polymeren Verbindung hin. Die charakteristische SH-Bande (ca. 2558 cm-1) ist nur schwach ausgeprägt, da die Infrarotbanden von Thiolgruppen generell eine sehr geringe Intensität aufweisen. Im FT-IR-Spektrum des Copolymers Poly(mercaptopropylmethyl-co-acryloxymethylpropyl)siloxan sind zusätzlich die charakteristischen IR-Banden der Acrylatgruppe (C=O-Banden bei 1727 cm⁻¹ und C=C-Banden bei 1637 und 1622 cm⁻¹) nachweisbar.

Im Zuge der TGA-Untersuchungen konnte gezeigt werden, dass die Homo- und Copolymere je nach Struktur bis zu einem Temperaturbereich von 240 °C bis 270°C stabil sind und dann einen mehrstufigen Abbau zeigen.

Um die Reaktivität der polymeren Vernetzungsmittel zu bestimmen wurde eine 2 Gew.-% Lösung Polyisoprenstandard in Chloroform hergestellt und mit 1phr Lucirin TPO-L und 5 phr des entsprechenden Thiols versetzt. Die Mischung wurde auf CaF₂-Plättchen gerakelt, das Lösungsmittel abgedampft und die dünnen Schichten anschließend mit einer UV-Lampe (Omni-Cure Series 1000; high pressure lamp, full power: 100 W von EXFO) belichtet. Nach unterschiedlichen Belichtungszeiten wurden IR-Spektren aufgenommen und die Abnahme der normierten C=C-Bande (835 cm⁻¹) über die Belichtungszeit aufgenommen. Im Vergleich zu dem kommerziell verfügbaren hochmolekularen Thiol Dipentaerythritolhexa(3-mercaptopropionat) (THIOCURE^{®} Di-PETMP, Bruno Bock Thiochemicals) weisen die Siloxanpolymere eine wesentlich höhere Reaktivität in der Vernetzung auf. Während bei Einsatz von THIOCURE^{®} Di-PETMP die relative Abnahme der C=C Banden nach einer Belichtungszeit von 150s etwa 5% beträgt kann bei Einsatz von Poly(mercaptopropylmethyl)siloxan eine Abnahme im Bereich von 12% erzielt werden.

Die Reaktivität der polymeren Vernetzer in der UV initiierten Thiol-En Reaktion wurde in weiteren Experimenten bestätigt. Dazu wurden die Vernetzungsmittel (1 phr) zusammen mit einem Photoinitiator (1 phr Lucirin TPO-L) in eine Polyisoprenstandardlösung (2 Gew.-% in Chloroform) gemischt und anschließend dünne Filme (40 µm) gerakelt, getrocknet, strukturiert belichtet und in Chloroform entwickelt. Ähnlich wie bei einem Negativresist werden durch die Thiol-En Reaktion die belichteten Bereiche der Schicht vernetzt und in der anschließenden Entwicklung in Chloroform konnten nur die nicht belichteten Bereiche gelöst und entfernt werden. Dieses Experiment wurde mit einem Mask Aligner bei einer sehr geringen Belichtungsdosis (~20 mW/cm², 80 s) durchgeführt, um den Einfluss der direkten C-C-Verknüpfung der Polymerketten durch die Photoinitiatorradikale möglichst gering zu halten. Die Ergebnisse zeigen, dass bereits eine geringe Konzentration (1 phr) der polymeren Vernetzer ausreicht, um eine sehr hohe ortsaufgelöste Vernetzung des Polyisoprenstandards zu erhalten. Die Ergebnisse bestätigen somit die hohe Reaktiviät und Effizienz der synthetisierten polymeren Vernetzer in der Thiol-En Reaktion.

Auf Grund seiner chemischen Struktur (hohe Konzentration an freien Si-OH-Gruppen) kann Poly(mercaptopropyl)siloxan im Zuge der Lagerung (auch unter Inertatmosphäre) über eine Kondensationsreaktion vernetzen. Die polymere Verbindung besitzt daher nur eine eingeschränkte Lagerbeständigkeit (ca. 1 Woche). UV vernetzte NR-Latexfilme (vor Alterung und Gamma-Sterilisation) mit 1 und 2phr Vernetzungsmittel weisen eine Reißfestigkeit von 12 MPa - 15 MPa auf.

Um mögliche Nachreaktionen (v.a. Vernetzung) während der Lagerung der polymeren Vernetzungsmittel möglichst gering zu halten wurden in weiteren Syntheseansätzen Disiloxanmonomere eingesetzt. Die polymere Verbindung ist durch die geringere Konzentration an freien Si-OH-Gruppen durch eine wesentlich höhere Lagerbeständigkeit gekennzeichnet und es wird auch über eine Lagerung von 1 Monat (unter Interatmosphäre) keine Änderung in der Viskosität beobachtet. Der Einfluss von unterschiedlichen Parametern (u.a. Reaktionszeit, Monomergehalt) in der Synthese auf die entsprechenden mechanischen Festigkeiten und Alterungsbeständigkeiten von NR-Latexfilmen wurde weiter untersucht.

Im ersten Schritt wurde die Synthese von Poly(mercaptopropylmethyl)siloxan bei einer konstanten Monomerkonzentration in der Reaktionsmischung (9% (w/v)) nach unterschiedlichen Reaktionszeiten (3, 6 und 9 Stunden) abgebrochen, das polymere Produkt aufgearbeitet und entsprechende Vernetzungsexperimente durchgeführt.

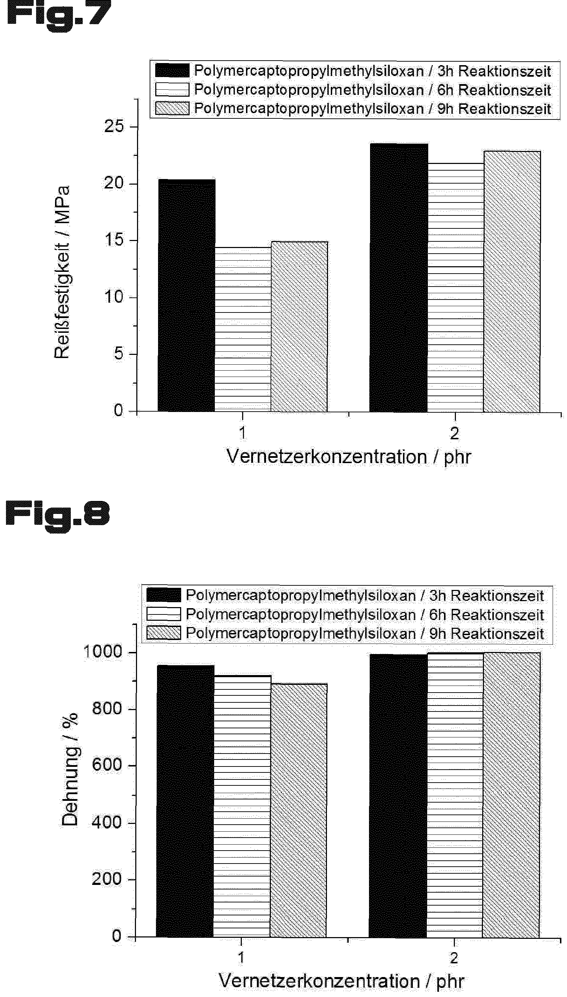

Bei einer Vernetzungsmittelkonzentration von 1 phr, weist das Polymer mit der geringeren Reaktionszeit (3 Stunden) die besseren mechanischen Festigkeiten auf (20 ± 2 MPa). Bei höheren Konzentrationen (2 phr) der polymeren Vernetzungsmittel kann jedoch nur ein geringfügiger Unterschied in den mechanischen Festigkeiten beobachtet werden und die Werte liegen in einem Bereich von 22 bis 24 MPa. Die Ergebnisse sind in den Fig. 7 bis 9 dargestellt.

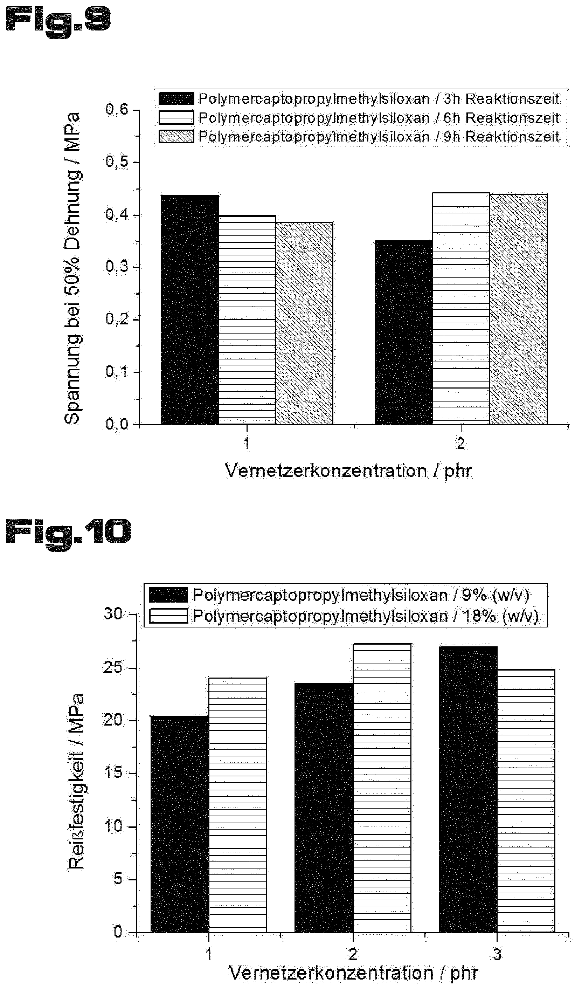

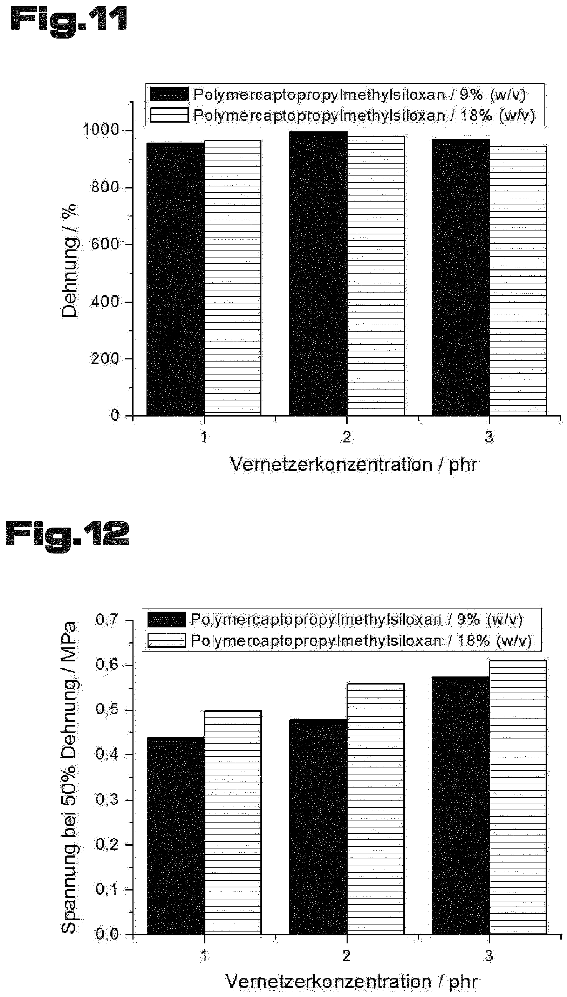

In weiterführenden Arbeiten wurde die Monomerkonzentration in der Synthese bei gleichbleibender Reaktionszeit von 3 Stunden variiert (9 und 18% (w/v)). Während bei geringeren Monomerkonzentrationen (9 (w/v)) eine Zunahme der Reißfestigkeit (von 20 auf 26 ± 2 MPa) mit zunehmender Vernetzerkonzentration in der Latexmischung (von 1 auf 3 phr) beobachtet werden kann, wird bei einer höheren Monomerkonzentration (18% (w/v)) ein Optimum bei 2 phr des Vernetzers in der Latexmischung erzielt (27 ± 2 MPa) erzielt. Die Ergebnisse dieser Untersuchung sind in den Figuren 10 bis 12 dargestellt.

Zusätzlich wurden NR-Latexfilme bei höheren Konzentrationen an polymeren Vernetzungsmitteln hergestellt, UV belichtet und der Einfluss der Vernetzungsmittelkonzentration auf die mechanischen Eigenschaften untersucht. Als polymeres Vernetzungsmittel wurde Poly(mercaptopropylmethyl)siloxan (Monomerkonzentration:18% (w/v); Reaktionszeit: 3h) gewählt. Die Ergebnisse lassen den Schluss zu, dass eine weitere Erhöhung der Vernetzungsmittelkonzentration von 3 phr auf 4 phr zu keiner signifikanten Verbesserung der Reißfestigkeiten führt. Es kann zwar eine Zunahme des Spannungswertes bei 50% Dehnung erzielt werden, was auf einen höheren Vernetzungsgrad hinweist, aber die Reißfestigkeiten bleiben im Bereich von 25 MPa.

In einem weiteren Schritt wurde ein polymeres Thiolvernetzungsmittel mit kürzerer Zwischengruppe (zwischen Thiolgruppe und polymerer Hauptkette) synthetisiert. Anstelle der Propylgruppe wurde eine Methylgruppe gewählt. Die Monomerkonzentration in der Synthese betrug 9% (w/v) und die Reaktionszeit drei Stunden. Auch diese polymere Verbindung ist lagerstabil und es können keine Viskositätsänderungen über eine Lagerzeit von einem Monat beobachtet werden.

Mit Poly(mercaptomethylmethyl)siloxan als Vernetzungsmittel können im Vergleich zu Poly(mercaptopropylmethyl)siloxan bei gleichen Vernetzerkonzentrationen (1 phr bzw. 2 phr) tendenziell höhere mechanische Festigkeiten bei der UV Vernetzung von NR-Latex erzielt werden. Die Reißfestigkeiten betragen für nicht sterile und nicht gealterte Filme bei einer Konzentration an Poly(mercaptopropylmethyl)siloxan von 1 phr ca. 23 MPa und bei 2 phr ca. 26 MPa. Die 50 % Moduln betragen für nicht sterile und nicht gealterte Filme bei einer Konzentration an Poly(mercaptopropylmethyl)siloxan von 1 phr ca. 0,45 MPa und bei 2 phr ca. 0,5 MPa.

Neben den mercapto-funktionellen Homopolymeren wurden auch Copolymere mit Acryloxypropylmethyleinheiten synthetisiert und als Vernetzungsmittel in der UV Vernetzung von NR-Latex eingesetzt. Durch die Acrylatgruppen als zweite Monomereinheit soll einerseits die Bildung von Disulfiden (als Nebenreaktion der Thiol-En Reaktion) unterbunden werden und andererseits eine reaktive Gruppe (Acrylate) für die Anbindung des polymeren Vernetzungsmittels an die Kautschukkette zur Verfügung stehen. Die Konzentration beider Monomere in der Synthese betrug gesamt 9% (w/v) und die Reaktionszeit drei Stunden. Auch diese polymere Verbindung ist lagerstabil und es können keine Viskositätsänderungen über eine Lagerzeit von einem Monat beobachtet werden. Mit dem Copolymer können im Vergleich zum entsprechenden Homopolymer (Poly(mercaptopropylmethyl)siloxan) bei gleicher Vernetzungsmittelkonzentration in der Latexmischung signifikant höhere Reißfestigkeiten (bis zu 30MPa) erzielt werden.

In weiteren Arbeiten wurde der Einfluss der Comonomerenzusammensetzung auf die mechanischen Eigenschaften untersucht. Hierbei wurde die Konzentration von 3-Acryloxypropylmethylsiloxan von 8,4 auf 16,8 %(mol/total mol) verdoppelt. Die Ergebnisse der Zugprüfung zeigen, dass mit 2 phr P(MPMS-co-APMS) eine Erhöhung der Acrylateinheiten in der Polymerkette mit einer leichten Verringerung von ca. 8% der mechanischen Eigenschaften verbunden ist.

Als Referenz wurde auch ein Acrylathomopolymer synthesiert (analog zu der voranstehend angegeben Synthese der Vernetzungsmittel) und als Vernetzungsmittel eingesetzt. Auf Grund der hohen Reaktivität der Acrylatgruppen wird eine photochemische Vernetzung über direkte C-C-Verknüpfung mit den Isopreneinheiten erzielt. Die erfolgreiche strukturierte Belichtung von Polyisoprenfilmen mit Poly(acryloxypropylmethyl)siloxan als Vernetzungsmittel lässt zwar auf eine ausreichend hohe Reaktivität in der UV-Vernetzung schließen, aber die mechanischen Eigenschaften von entsprechenden NR-Latexfilmen (mit 1 phr und 2 phr Vernetzungsmittel) liegen deutlich niedriger (14 bis 17 MPa) im Vergleich zu den mercapto-funktionellen Homo- und Copolymeren. Zusätzlich sind die Filme durch eine unzureichende Alterungsbeständigkeit (7 Tage Heißluftalterung bei 70°C) gekennzeichnet (<3 MPa und starke Gelbfärbung).

In weiteren Experimenten wurde der Einfluss der Vor- bzw. Nachvernetzung auf die Vernetzung von NR-Latex und IR-Latex mit Poly(mercaptomethylmethyl) siloxan als polymeres Vernetzungsmittel untersucht.

### Vorvernetzung:

Die synthetisierten polymeren Vernetzungsmittel (0,5 phr) wurden mit Lucirin TPO-L (0,5 phr) in deionisiertem Wasser mit Tween 20 (0,1 phr) emulgiert und anschließend dem NR-Latex (40 drc.) bzw. dem IR-Latex (40 drc., Kraton) zugegeben. Die Latexmischung wurde 2 Stunden bei Raumtemperatur gerührt. Anschließend wurde die jeweilige Latexmischung in eine Petrischale gegossen (ca. 1 mm Schichtdicke) und mit einem UV-Strahler von Fusion UV Systems Inc. belichtet. Die NR-Latexmischungen wurden unter Luft mit einem Ga-dotierten Hg-Strahler bei einer Lampenleistung von 60% und einer Bandgeschwindigkeit von 3,5 m/min in vier Durchgängen bestrahlt (entspricht einer Strahlungsdosis von 20,8 J/cm²). Die IR-Latexmischungen wurden unter Luft mit einem Ga-dotierten Hg-Strahler bei einer Lampenleistung von 60% und einer Bandgeschwindigkeit von 3,5 m/min in zwei Durchgängen bestrahlt (entspricht einer Strahlungsdosis von 10,4 J/cm²).

Bei der Herstellung von vorvernetzten Filmen (ohne anschließende Nachvernetzung) wurde die Latexmischung nach der Vorvernetzung mit dem Alterungsschutzmittel (0,5 phr Ionol LC) versetzt und 2 Stunden bei Raumtemperatur gerührt. Anschließend erfolgte die Tauchung der Latexfilme mittels Koagulationstauchverfahren. Hierbei werden nachfolgende Arbeitsschritte durchgeführt:
- Waschen der Keramikformen und Entfetten mit Aceton
- Vorwärmen der Keramikformen für mindestens 10 Minuten im Trockenschrank bei 120 °C
- Tauchen der Form für 30 Sekunden in das Koagulationsbad bei 70 °C
- Trocknen der Form für mindestens 1 Minute im Trockenschrank bei 120 °C
- Tauchen der Form in die NR-Latexmischung für 20 Sekunden
- Trocknen für 20 Minuten im Trockenschrank bei 120 °C
- Abziehen

### Nachvernetzung:

Wahlweise wurde auch eine Nachvernetzung durchgeführt. Hierbei wurden die jeweiligen Latexmischungen (vorvernetzt bzw. nicht vorvernetzt) mit einer Emulsion bestehend aus dem synthetisierten polymeren Vernetzungsmittel (2 phr), Lucirin TPO-L (1 phr), deionisiertem Wasser (2 phr) und Tween 20 (0,1 phr), versetzt. Anschließend wurde die Latexmischung mit einem Alterungsschutzmittel (0,5 phr Ionol LC) versetzt und 2 Stunden bei Raumtemperatur gerührt.

Entsprechende Filme wurden mit Hilfe des Koagulationstauchverfahrens hergestellt und die Nachvernetzung erfolgt im Zuge einer UV-Belichtung der getrockneten Filme (Post-Curing) mit einem UV-Strahler von Fusion UV Systems Inc. Sowohl NR-Latexfilme als auch IR-Latexfilme wurden unter Luft mit einem Ga-dotierten Hg-Strahler bei einer Lampenleistung von 60% und einer Bandgeschwindigkeit von 3,5 m/min in drei Durchgängen bestrahlt (entspricht einer Strahlungsdosis von 15,6 J/cm²).

Bei der photochemischen Vernetzung des NR-Latex zeigte sich, dass die höchsten mechanische Festigkeiten durch eine Nachvernetzung erzielt werden können. Die Reißfestigkeit der NR-Latexfilme (nicht steril, nicht gealtert) betrug ca. 22,5 MPa für die vorvernetzte Probe, ca. 18 MPa für die vor- und nachvernetzte Probe und ca. 25 MPa für die ausschließlich nachvernetzte Probe. Überraschend ist dieses Ergebnis insofern, als die vor- und nachvernetzte Probe die geringste Reißfestigkeit aufwies.

Ein vergleichbarer Trend wird auch bei der UV Vorvernetzung von IR-Latex beobachtet. Hier liegt die Reißfestigkeit von vorvernetzten IR-Latexfilmen im Bereich von 3,5 MPa. Im Gegensatz zu NR-Latexfilmen gelingt aber durch eine kombinierte Vor- und Nachvernetzung eine deutliche Steigerung der Reißfestigkeiten auf 16MPa.

Zur weiteren Evaluierung der polymeren Vernetzungsmittel wurden weitere Mercaptopolymere synthetisiert. Dazu wurde für eine kontrolliertere Polymerisation (Herstellung von Polymeren mit niedrigerem Polydispersitätsindex) die Herstellung von Poly(mercaptomethylmethyl)siloxan und Poly(mercaptopropylmethyl-co-acryloxypropylmethyl)siloxan in Gegenwart von 2 phr bzw. 3 phr Methoxytrimethylsilan als Terminierungsreagenz durchgeführt. Die Synthese erfolgte analog zu voranstehend beschriebener Synthese, wobei zusätzlich 2 phr bzw. 3 phr Methoxytrimethylsilan (Sigma-Aldrich) der Reaktionsmischung zugegeben wurde.

Die Reißfestigkeiten der UV nachvernetzten NR-Latexfilmen (nicht steril, nicht gealtert) liegen durchgehend zwischen 25 MPa und 27 MPa.

Zur Bestimmung der extrahierbaren Vernetzungsmittelkonzentration wurden nachvernetzte NR-Latexfilme im Zuge einer Soxhletextraktion (10 Stunden / Toluol) extrahiert. Das Lösungsmittel wurde mittels Rotavapor abgezogen und der Extrakt im Vakuumtrockenschrank bis zur Gewichtskonstanz bei 35°C und 100 mbar getrocknet.

Mittels C/H/N/S wurde in einer Dreifachbestimmung die extrahierbaren S-Verbindungen (Thiole und Proteine des NR-Latex) bestimmt.

Die Ergebnisse der Elementaranalyse zeigen eine deutlich geringere Extrahierbarkeit (75%) des Vernetzungsmittels im Vergleich zu niedermolekularen Thiolen (wie z.B. Trimethylolpropan-trimercaptopropionat, TMPMP).

**Tabelle 3 - S-Konzentration im Extrakt von UV nachvernetzten NR-Latexfilmen**

| Probe | Photoinitiator | Thiol | Extrahierbarer S-Gehalt / mgS/gLatex |
|---|---|---|---|
| Referenz | Lucirin TPO-L (1phr) | TMPMP (2phr) | 2,074 |
| Homopolymer | Lucirin TPO-L (1phr) | Poly(mercaptopropylmethyl methyl)siloxan (2phr) | 0,534 |
| Copolymer | Lucirin TPO-L (1phr) | Poly(mercaptopropylmethyl-co-acryloxypropylmethyl)siloxan (2phr) | 0,522 |

In den nachfolgenden Ausführungsbeispielen soll gezeigt werden, dass die thermische Vernetzung von XNBR-Latexfilmen nicht nur mit polaren, wasserlöslichen Epoxidvernetzungsmitteln sondern auch mit unpolareren Epoxidderivaten gelingt.

### Beispiel A - Vernetzung mit Bisphenol A Diglycidylether

3 phr Bisphenol A Diglycidylether (Huntsman) werden in 6 phr deionisiertem Wasser mit 0,3 phr Tween 20 emulgiert. Anschließend wird die Emulsion der Latexmischung (pH = 10,2; ~25 drc.) zugegeben und die Latexmischung für 60 Minuten bei Raumtemperatur gerührt. Die Filme werden analog zur beschriebenen Durchführung hergestellt und die thermische Vernetzung erfolgt im Zuge der Trocknung der Filme im Umlufttrockenschrank.

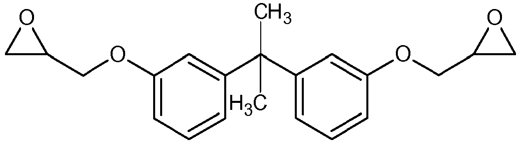

### Bisphenol A Diglycidylether

### Beispiel B - Vernetzung mit einem hydrierten Bisphenol A Diglycidylether

Die Herstellung erfolgt analog zu Beispiel A - nur anstelle des Bisphenol A Diglycidylethers werden 3 phr bzw. 5 phr eines hydrierten Bisphenol A Diglycidylethers (EPALLOY^{®}5000 und EPALLOY^{®}5001 von CVC Thermoset Specialities) eingesetzt.

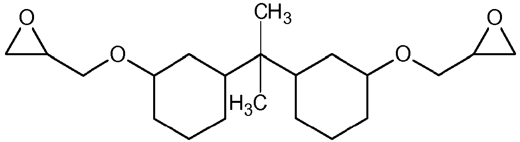

### Hydrierter Bisphenol A Diglycidylether

### Beispiel C - Vernetzung mit einem Hexahydrophtalsäurediglycidylether

Die Herstellung erfolgt analog zu Beispiel A - nur anstelle des Bisphenol A Diglycidylethers wird ein Hexahydrophtalsäurediglycidylether (3 phr und 5 phr EPALLOY^{®}5200 von CVC Thermoset Specialities) eingesetzt

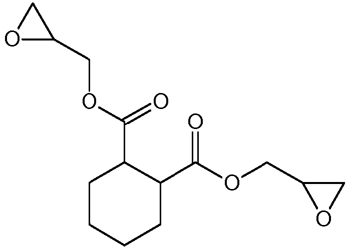

### Hexahydrophtalsäurediglycidylether

### Beispiel D - Vernetzung mit einem 1,4-Cyclohexandimethanoldiglycidylether

Die Herstellung erfolgt analog zu Beispiel A - nur anstelle des Bisphenol A Diglycidylethers wird ein 1,4-Cyclohexandimethanoldiglycidylether (3 phr und 5 phr ERISYS^{™} GE 22 von CVC Thermoset Specialities) eingesetzt.

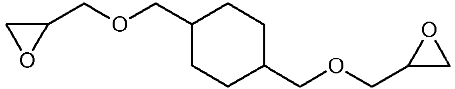

### 1,4-Cyclohexandimethanoldiglycidylether

Die gemessenen mechanischen Eigenschaften der gemäß den Beispielen A-D vernetzten XNBR-Latices sind in Tabelle 4 zusammengefasst.

**Tabelle 4 - Mechanische Eigenschaften von thermisch vernetzten XNBR-Latexfilmen bei Einsatz von unterschiedlichen Epoxiden**

| Epoxidvernetzer | Konzentration Vernetzer [phr] | Reißfestigkeit [MPa] | Spannung [%] | Spannung bei 50% Dehnung [MPa] |
|---|---|---|---|---|
| Bisphenol A Diglycidylether | 3 | 42,2 | 700 | 1,58 |
| EPALLOY^{®}5000 | 3 | 39,3 | 700 | 1,53 |
| EPALLOY^{®}5000 | 5 | 39,9 | 670 | 1,53 |
| EPALLOY^{®}5001 | 3 | 36,9 | 680 | 1,62 |
| EPALLOY^{®}5001 | 5 | 38,4 | 670 | 1,59 |
| EPALLOY^{®}5200 | 3 | 36,8 | 690 | 1,59 |
| EPALLOY^{®}5200 | 5 | 38,4 | 690 | 1,46 |
| ERISYS^{™} GE22 | 3 | 34,0 | 680 | 1,60 |
| ERISYS^{™} GE22 | 5 | 34,9 | 670 | 1,48 |

Die Ausführungsbeispiele beschreiben mögliche Ausführungsvarianten des Verfahrens, auch diverse Kombinationen der einzelnen Ausführungsvarianten untereinander möglich sind.

## Patentansprüche

1. Verfahren zum Herstellen eines Prophylaxeartikels, insbesondere eines Handschuhs, aus einem Dienkautschuk und/oder einem carboxylierten Dienkautschuk, nach dem auf eine Form zumindest eine Schicht aus einem Dienlatex und/oder einem carboxylierten Dienlatex aufgebracht wird, und der Dienlatex und/oder der carboxylierte Dienlatex mit einem Vernetzungsmittel vernetzt wird, wobei als Vernetzungsmittel ein mehrfach funktionelles Monomer und/oder Polymer mit zwei oder mehr Wiederholungseinheiten verwendet wird, das dem Dienlatex und/oder dem carboxylierten Dienlatex zugesetzt und in diesem gelöst oder in diesem emulgiert bzw. dispergiert wird, wobei das Vernetzungsmittel dem Dienlatex und/oder dem carboxylierten Dienlatex in einem Anteil von 1 phr bis 10 phr bezogen auf die Gesamtzusammensetzung des Dienlatex und/oder des carboxylierten Dienlatex zugesetzt wird und ausgewählt wird aus einer Gruppe bestehend aus mehrfachfunktionellen Epoxiden, mehrfachfunktionellen Silanen, mehrfachfunktionellen polymeren Thiolen, sowie Mischungen daraus, **dadurch gekennzeichnet, dass** als Vernetzungsmittel ausschließlich das mehrfach funktionelle Monomer und/oder Polymer mit zumindest zwei oder mehr Wiederholungseinheiten verwendet wird, und dass der pH-Wert des Dienlatex und/oder des carboxylierten Dienlatex auf einen Wert von grösser/gleich 9 eingestellt wird.

2. Verfahren nach Anspruch 1, **dadurch gekennzeichnet, dass** die Vernetzung der Dienlatexmoleküle und/oder carboxylierten Dienlatexmoleküle thermisch und/oder photochemisch mittels ultravioletter Strahlung durchgeführt wird.

## Claims

1. Process for producing a prophylactic article, in particular a glove, from a diene rubber and/or a carboxylated diene rubber, in which at least one layer of a diene latex and/or a carboxylated diene latex is applied to a mold, and the diene latex and/or the carboxylated diene latex is crosslinked with a crosslinking agent, wherein a multifunctional monomer and/or polymer with two or more repeating units is used as crosslinking agent, which is added to the diene latex and/or the carboxylated diene latex and is dissolved or emulsified respectively dispersed therein, wherein the crosslinking agent is added to the diene latex and/or the carboxylated diene latex in a proportion of 1 phr to 10 phr, based on the total composition of the diene latex and/or the carboxylated diene latex, and is selected from a group consisting of multifunctional epoxides, multifunctional silanes, multifunctional polymeric thiols, and mixtures thereof, **characterized in that** only the multifunctional monomer and/or polymer with at least two or more repeating units is used as crosslinking agent, and that the pH value of the diene latex and/or the carboxylated diene latex is adjusted to a value greater than or equal to 9.

2. Process according to claim 1, **characterized in that** the crosslinking of the diene latex molecules and/or carboxylated diene latex molecules is carried out thermally and/or photochemically by ultraviolet radiation.

## Revendications

1. Procédé de fabrication d'un article prophylactique, en particulier un gant, à partir d'un caoutchouc diénique et/ou d'un caoutchouc diénique carboxylé, dans lequel au moins une couche d'un latex diénique et/ou d'un latex diénique carboxylé est appliquée sur un moule, et le latex diénique et/ou le latex diénique carboxylé est réticulé avec un agent de réticulation, dans lequel un monomère multifonctionnel et/ou un polymère comportant deux ou plusieurs unités répétitives est utilisé comme agent de réticulation, qui est ajouté au latex diénique et/ou au latex diénique carboxylé et y est dissous ou émulsionné ou dispersé, dans lequel l'agent de réticulation est ajouté au latex de diène et/ou au latex de diène carboxylé dans une proportion de 1 phr à 10 phr, sur la base de la composition totale du latex de diène et/ou du latex de diène carboxylé, et est choisi dans un groupe constitué d'époxydes multifonctionnels, des silanes multifonctionnels, des thiols polymères multifonctionnels et leurs mélanges, **caractérisé en ce que** seul le monomère multifonctionnel et/ou le polymère comportant au moins deux ou plusieurs unités répétitives est utilisé comme agent de réticulation, et **en ce que** le pH du latex de diène et/ou du latex de diène carboxylé est ajusté à une valeur supérieure ou égale à 9.

2. Procédé selon la revendication 1, **caractérisé en ce que** la réticulation des molécules de latex diénique et/ou des molécules de latex diénique carboxylé est effectuée thermiquement et/ou photochimiquement par rayonnement ultraviolet.
